# EUROPEAN PATENT APPLICATION

(11) **EP 2 305 234 A1**
(43) Date of publication of application: **06.04.2011**
(21) Application number: 09766696.0
(22) Date of filing: 18.06.2009
(51) Int. Cl.: A61K 31/165, A61K 31/201, A61K 31/381, A61K 31/405, A61K 31/4406, A61K 31/4418, A61P 1/16, A61P 31/14, A61P 35/00

(54) **PHARMACEUTICAL COMPOSITION FOR TREATMENT OR PREVENTION OF HBV INFECTION**

(30) Priority: 19.06.2008 JP 2008160601
(71) Applicant: Public University Corporation Nagoya City University, Mizuho-ku Nagoya-shi Aichi 467-8601 (JP); Chugai Seiyaku Kabushiki Kaisha, Kita-ku Tokyo 115-8543 (JP)
(72) Inventor: MIZOKAMI, Masashi, Nagoya-shi Aichi 467-8601 (JP); TANAKA, Yasuhito, Nagoya-shi Aichi 467-8601 (JP); SUGIYAMA, Masaya, Nagoya-shi Aichi 467-8601 (JP); SUDOH, Masayuki, Kamakura-shi Kanagawa 247-8530 (JP)
(74) Representative: Jaenichen, Hans-Rainer
(86) International application number: PCT/JP2009/061087
(87) International publication number: WO 2009/154248

(57) **Abstract**

First, the present inventors assessed the effect of the compound represented by formula (III) below on Huh-7 cells infected with HBV, and demonstrated that the compound alone had an anti-HBV effect *in vitro.*

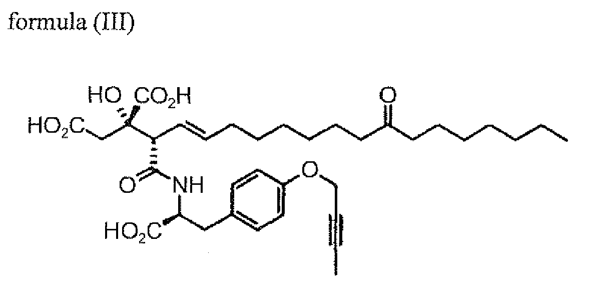

Then, the present inventors revealed that the HBV replication-suppressing effect of PEG-IFN is enhanced in chimeric mice having a human liver infected with genotype C or A HBV when PEG-IFN is used in combination with the compound represented by formula (III) above. The present inventors also revealed that the HBV replication-suppressing effect of Entecavir is enhanced in chimeric mice having a human liver infected with genotype C HBV (wild-type and Entecavir-resistant strains) when Entecavir is used in combination with the compound represented by formula (III) above. In addition, the present inventors revealed that the compound represented by formula (III) above exerts the anti-HBV effect not only against wild-type HBV strains but also against Entecavir- and/or Lamivudine-resistant HBV strains.

## Description

### Technical Field

The present invention relates to novel pharmaceutical compositions for treating or preventing HBV infection.

### Background Art

Hepatitis B virus (HBV) is an incomplete double-stranded DNA virus belonging to genus *Hepadnaviridae.* HBV infection is a major health problem worldwide, and the number of infected people around the world is 350 million. The infection rate in Japan is thought to be around 1 %, with an estimated 1,500,000 infected people, approximately. The infection rate is very high, mainly in Southeast Asia. Thus, HBV infection remains a serious problem. Acute hepatitis B remits spontaneously in most cases, but may infrequently cause fulminant hepatitis. Once acute hepatitis B becomes chronic, it can progress from chronic hepatitis to cirrhosis, and then to hepatocellular carcinoma in some clinical cases. In Japan, HBV infection was previously caused mostly by maternal infection; in recent years however, a vaccine is administered to babies born of HBs antigen-positive mothers, making new cases of maternal infection rare. Recently however, acute B hepatitis is on the rise among young people as a sexually-acquired infection. This acute B hepatitis infection becomes chronic (Non-patent Document 1) in some cases, resulting in persistent liver function abnormalities, which can then lead to chronic hepatitis, cirrhosis, and hepatocellular carcinoma.

Two types of clinical conditions are seen in patients persistently-infected with HBV: cases showing rapid changes in the transaminase level who develop cirrhosis at an early stage, and even progress to hepatocellular carcinoma; and cases in which the transaminase level is maintained within a normal range and who hardly develop pathological conditions. The reason for such a significant difference in the host pathological condition still remains to be clarified. However, It is well conceivable that both viral and host factors are involved in the difference. HBV genotype has been recently drawing attention as a viral factor (Non-patent Document 2). HBV genotypes are grouped into A to H, and regional specificities and clinical differences have been reported (Non-patent Document 3).

Current major therapeutic methods for HBV infection are: interferon (IFN) therapy and nucleic acid analog preparations (Lamivudine [LMV], Adefovir [ADV], and Entecavir [ETV]). The complete response rate of IFN in HBe antigen-positive cases is 1/3 or less, which differs depending on the period of administration. The HBe antigen response rate of LMV, a nucleic acid analog preparation, can be increased by prolonging the period of administration. However, this therapy has the issues of having a high likelihood of hepatitis exacerbating after discontinuation of administration; and emergence of drug resistance strains. Exacerbated hepatitis due to emergence of LMV-resistant virus can be treated with IFN or other nucleic acid analog preparations (ADV or ETV). Of these treatments, IFN therapy can control hepatitis to some extent; however, it has the following problems: development of adverse effects and limitations on the period of administration. ADV and ETV are used for LMV-resistant virus, but these are also nucleic acid analogs, and viruses resistant to ADV or ETV have already emerged. In any case, the issue of emergence of resistant viruses remains to be solved and there is a need to develop antiviral agents that do not develop resistant virus.

Of the compounds represented by formula (I) below, some of them are reported to be useful as therapeutic agents against fungal infection and immunological disorders (Patent Document 1 (WO98/56755));
some of them are reported to have anti-HCV activity (Patent Documents 2 (WO2004/071503) and 3 (WO2005/005372));
some of them are reported to produce an anti-HCV effect by using in combination with interferons (Patent Document 4 (WO2007/132882)); and
some of them are reported to yield not only an anti-HCV effect but also an anti-HIV effect and an anti-influenza virus effect by blocking the process of sphingomyelin biosynthesis (Patent Document 5 (WO2006/016657)). However, it has neither been described nor suggested that the compounds have an anti-HBV effect.

Prior art document information related to the present invention is provided below.

### [Prior Art Documents]

[Patent Documents]
[Patent Document 1] WO98/56755
[Patent Document 2] WO2004/071503
[Patent Document 3] WO2005/005372
[Patent Document 4] WO2007/132882
[Patent Document 5] WO2006/016657
[Non-patent Documents]
[Non-patent Document 1] Ozasa A, Tanaka Y, Orito E and Mizokami M. Influence of Genotypes and Precore Mutations on Fulminant or Chronic Outcome of Acute Hepatitis B Virus Infection. Hepatology. 2006; 44: 326-334.
[Non-patent Document 2] Miyakawa Y, Mizokami M. Classifying hepatitis B virus genotypes. Intervirology 2003; 46: 329-338.
[Non-patent Document 3] Orito E, Mizokami M, Sakugawa H, et al. A case-control study for clinical and molecular biological differences between hepatitis B viruses of genotypes B and C. Japan HBV Genotype Research Group. Hepatology 2001; 33: 218-223.

### Disclosure of the Invention

### [Problems to be Solved by the Invention]

The present invention was achieved in view of the circumstances described above. An objective of the present invention is to provide novel pharmaceutical compositions for treating or preventing HBV infection.

### [Means for Solving the Problems]

The present inventors conducted dedicated studies to achieve the above-described objective. First, the present inventors tested the anti-HBV effect of the compound represented by formula (III) below on HBV-infected Huh-7 cells. The result showed that the level of HBV RNA was decreased after addition of the above-described compound, and thus the compound alone has the anti-HBV effect *in vitro.*

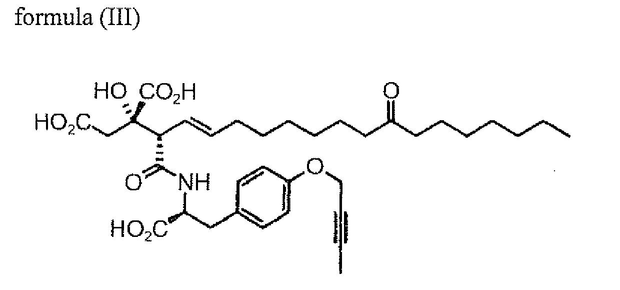

Then, the anti-HBV effect of the compound represented by formula (III) above and PEGylated interferon (PEG-IFN) was assessed using chimeric mice having human liver infected with genotype C or A HBV. The result showed that in both chimeric mice having human liver infected with genotype C HBV and those with genotype A HBV, the effect of PEG-IFN to suppress HBV replication was enhanced by using in combination with the compound represented by formula (III) above.

Furthermore, the anti-HBV effect of the compound represented by formula (III) above and Entecavir was assessed using chimeric mice having human liver infected with genotype C HBV (wild-type and Entecavir-resistant strains). The result showed that the effect of Entecavir to suppress HBV replication was enhanced by using in combination with the compound represented by formula (III) above.

In addition, it was demonstrated that, when used in combination with PEG-IFN or Entecavir, or even when used alone, the compound represented by formula (III) above produced the anti-HBV effect not only against wild-type HBV strain but also against Entecavir- and/or Lamivudine-resistant HBVs.

Specifically, the present inventors discovered that the compound represented by formula (III) could be used alone as a novel therapeutic agent for HBV infection and that a higher effect of inhibiting HBV replication was obtained when the compound represented by formula (III) above was used in combination with interferons or a nucleic acid analog. Thus, the present inventors completed the present invention.

More specifically, the present invention provides:
[1] a pharmaceutical composition for treating or preventing HBV infection, which comprises as an active ingredient a compound represented by: 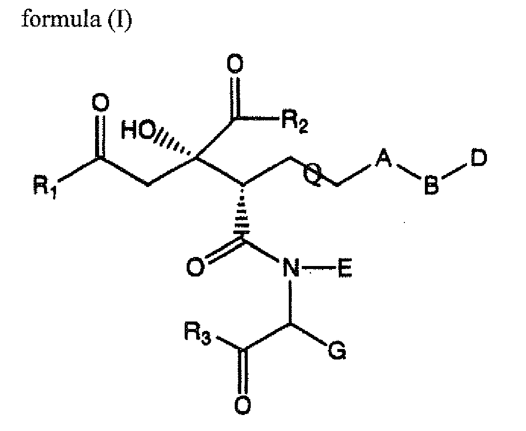 or a pharmaceutically acceptable salt thereof;
   wherein
   A represents -(CH₂)ₙ-; wherein n represents an integer from 0 to 10;
   B represents -CH₂-, -(C=O)-, -CH(OH)-, -CH(NH₂)-, or -C(=NOR)-; wherein R represents a hydrogen atom, a linear or branched alkyl group of 1 to 8 carbon atoms, which is optionally substituted with an amino group that is optionally mono- or di-substituted with a linear or branched alkyl group of 1 to 4 carbon atoms;
   D represents -(CH₂)ₘ-R'; wherein m represents an integer from 0 to 10; R' represents a hydrogen atom, a linear or branched alkyl group, a linear or branched alkynyl group, a linear or branched alkenyl group, a cycloalkyl group, a cycloalkenyl group, an optionally substituted heterocyclic group, an optionally substituted aryl group, an optionally substituted heteroaryl group, -OX group (wherein X represents a hydrogen atom, a linear or branched alkyl group, a linear or branched alkynyl group, a linear or branched alkenyl group, a cycloalkyl group, or an optionally substituted aryl group), or a halogen atom;
   E represents a hydrogen atom or a linear or branched alkyl group;
   G represents -(CH₂)ₚ-J; wherein p represents an integer from 0 to 4; J represents a hydrogen atom, an OH group, an SH group, a methylthio group, a carboxyl group, a carbamoyl group, an amino group, a guanidino group, a linear or branched alkyl group, a cycloalkyl group, a linear or branched alkynyl group, a linear or branched alkenyl group, an optionally substituted aryl group, an optionally substituted heterocyclic group, or an optionally substituted heteroaryl group;
   bond Q represents a single bond or double bond; and
   R¹, R², and R³ are the same or different and each represents a hydroxyl group, an amino group which is optionally mono- or di-substituted by a linear or branched alkyl group having 1 to 4 carbon atoms, -OL, a linear or branched alkyl group, a linear or branched alkenyl group, or a linear or branched alkynyl group; wherein L represents a linear or branched alkyl group, a linear or branched alkenyl group, or a linear or branched alkynyl group;
[2] the pharmaceutical composition of [1], wherein the compound is represented by any one of: 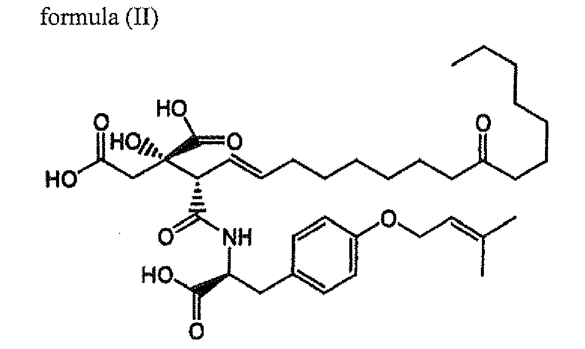 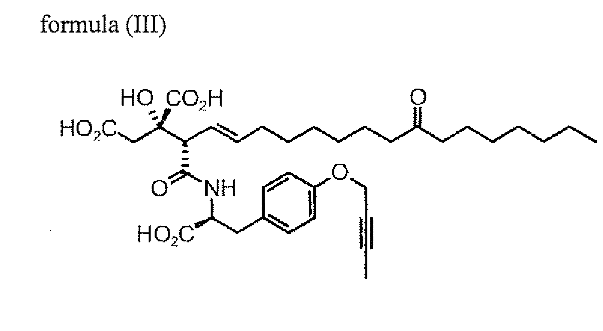 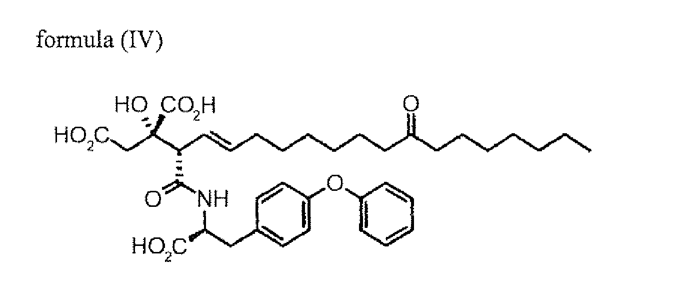 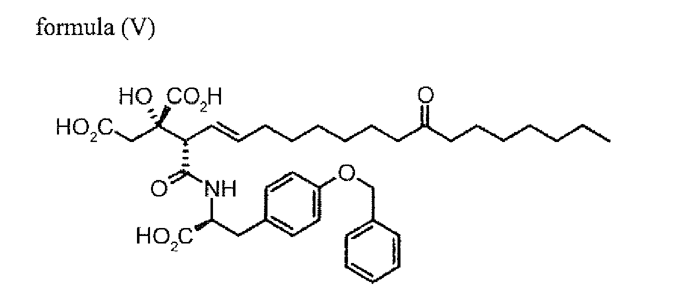 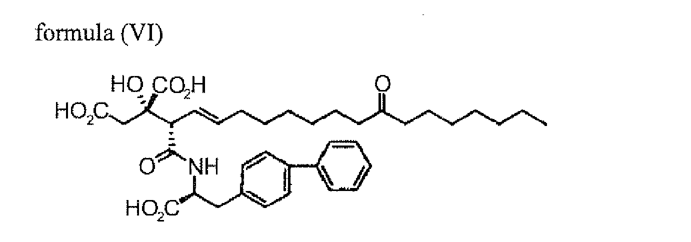 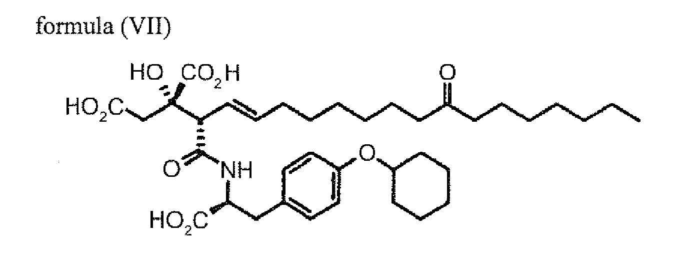 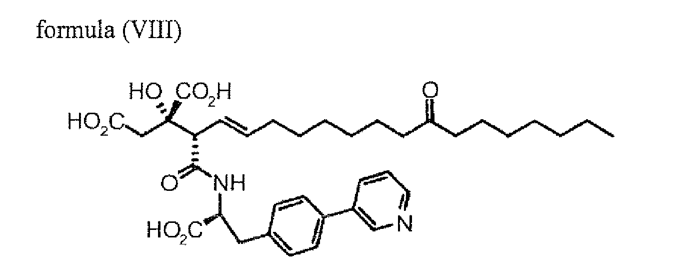 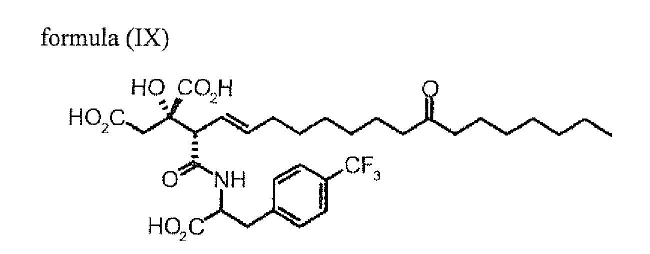 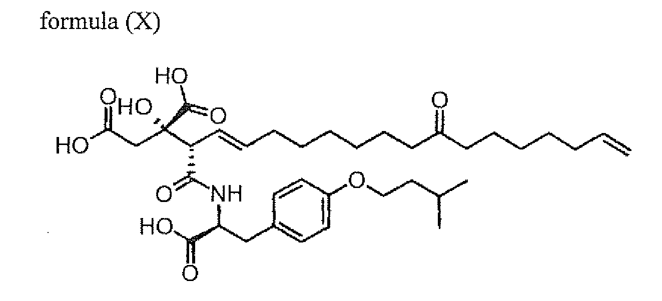 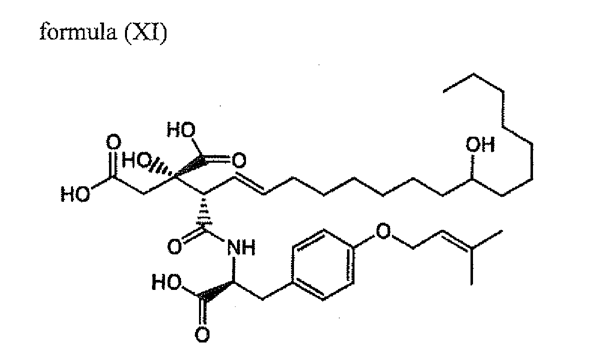 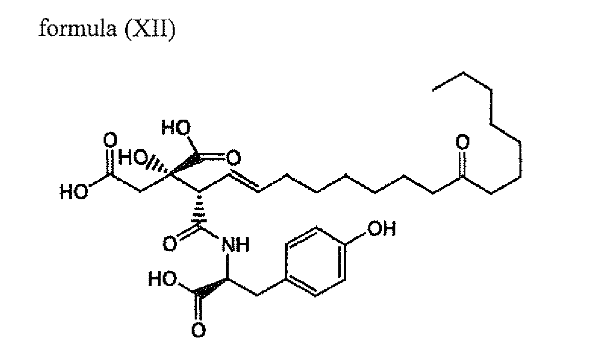 or a pharmaceutically acceptable salt thereof;
[3] the pharmaceutical composition of [1] or [2], wherein the HBV infection is hepatitis B, cirrhosis, or liver cancer;
[4] a pharmaceutical composition for treating or preventing HBV infection, which comprises a compound represented by formula (I) above in combination with one or more other anti-HBV agents;
[5] a pharmaceutical composition for treating or preventing HBV infection, which is used in combination with other anti-HBV agent(s), and which comprises as an active ingredient a compound represented by formula (I) above;
[6] a method for treating or preventing HBV infection, which comprises the step of administering at a therapeutically effective dose a compound represented by formula (I) above, or a pharmaceutically acceptable salt thereof, to a subject;
[7] use of a compound represented by formula (I) above or a pharmaceutically acceptable salt thereof in preparation of a pharmaceutical composition for treating or preventing HBV infection; and
[8] a compound represented by formula (I) above or a pharmaceutically acceptable salt thereof for use in a method for treating or preventing HBV infection.

### Brief Description of the Drawings

Fig. 1A is a graph showing the effect of an agent on Huh-7 cells infected with genotype C_AT HBV (wild-type strain).
Fig. 1B is a graph showing the effect of an agent on Huh-7 cells infected with various HBV strains.
   A: highly Lamivudine-resistant strain, 50% propagation suppression = 3.4 µM;
   B: Entecavir-resistant strain, 50% propagation suppression = 3.7 µM;
   C: Lamivudine-resistant strain, 50% propagation suppression = 3.6 µM; Wild: wild-type strain, 50% propagation suppression = 3.2 µM.
Fig. 2 is a graph showing the effect of an agent on genotype C_AT HBV (wild-type strain).
Fig. 3 is a graph showing the effect of an agent on genotype A HBV (wild-type strain).
Fig. 4 is a graph showing the effect of an agent on genotype C_AT HBV (wild-type strain).
Fig. 5 is a graph showing the effect of an agent on genotype C HBV (Entecavir-resistant strain).

### Mode for Carrying Out the Invention

The present invention relates to novel pharmaceutical compositions for treating or preventing HBV infection, which comprise as an active ingredient a compound represented by formula (I) or a pharmaceutically acceptable salt thereof.

The compound represented by formula (I) below has an anti-HBV activity. Thus, the compound represented by formula (I) below and pharmaceutically acceptable salts thereof are useful as an agent for treating or preventing HBV infection. 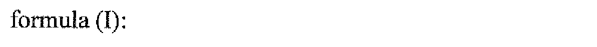 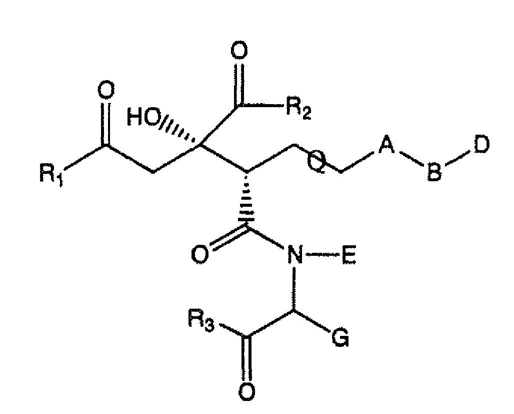 wherein
A represents -(CH₂)ₙ-, wherein n is an integer from 0 to 10;
B represents -CH₂-, -(C=O)-, -CH(OH)-, -CH(NH₂)-, or -C(=NOR)-, wherein R represents a hydrogen atom, or a linear or branched alkyl group of one to eight carbon atoms, which is optionally substituted with an amino group that is optionally mono- or di-substituted with a linear or branched alkyl group of one to four carbon atoms;
D represents -(CH₂)ₘ-R', wherein m is an integer from 0 to 10, and R' represents a hydrogen atom, a linear or branched alkyl group, a linear or branched alkynyl group, a linear or branched alkenyl group, a cycloalkyl group, an optionally substituted heterocyclic group, an optionally substituted aryl group, an optionally substituted heteroaryl group, a -OX group (wherein X denotes a hydrogen atom, a linear or branched alkyl group, a linear or branched alkynyl group, a linear or branched alkenyl group, a cycloalkyl group, or an optionally substituted aryl group), or a halogen atom;
E represents a hydrogen atom or a linear or branched alkyl group;
G represents -(CH₂)ₚ-J, wherein p is an integer from 0 to 4, and J represents a hydrogen, an OH group, an SH group, a methylthio group, a carboxyl group, a carbamoyl group, amino group, a guanidino group, a linear or branched alkyl group, a cycloalkyl group, a linear or branched alkynyl group, a linear or branched alkenyl group, an optionally substituted aryl group, an optionally substituted heterocyclic group, or an optionally substituted heteroaryl group;
bond Q represents a single bond or a double bond; and
R¹, R², and R³ are the same or different and represent a hydroxyl group, an amino group that is optionally mono- or di-substituted with a linear or branched alkyl group of one to four carbon atoms, -OL, a linear or branched alkyl group, a linear or branched alkenyl group, or a linear or branched alkynyl group, wherein L represents a linear or branched alkyl group, a linear or branched alkenyl group, or a linear or branched alkynyl group.

In the present invention, unless specifically defined herein, the linear or branched alkyl group means a linear or branched hydrocarbon group of one to twelve carbon atoms, and preferably means a linear or branched hydrocarbon group of one to seven carbon atoms. Examples include a methyl group, ethyl group, propyl group, isopropyl group, *n*-butyl group, isobutyl group, *t*-butyl group, pentyl group, and heptyl group. The cycloalkyl group means a cyclic hydrocarbon group of three to eight carbon atoms. Examples include a cyclopentyl group, cyclohexyl group, cycloheptyl group, and cyclohexenyl group. The linear or branched alkenyl group means a linear or branched hydrocarbon group of two to eight carbon atoms, which comprises at least one double bond. Examples include a vinyl group, 1-propenyl group, allyl group, 2-butenyl group, and 2-ethenyl-2-butenyl group. The linear or branched alkynyl group means a linear or branched hydrocarbon group of two to eight carbon atoms, which comprises at least one triple bond. Examples include an ethynyl group, 1-propynyl group, 2-propynyl group, 1-butynyl group, 2-butybyl group, 3-butynyl group, 2-pentynyl group, 3-pentynyl group, 4-pentynyl group, 2-hexynyl group, 4-hexynyl group, 2-decynyl group, and 6,6-dimethyl-hepta-2,4-diyn-1-ylgroup.

The heterocyclic group described herein means a four- to six-membered monocyclic or seven- to ten-membered bicyclic group (preferably a monocyclic group), which comprises one to four (preferably one or two) heteroatoms that are individually selected from nitrogen, sulfur, and oxygen atoms as ring members, and may comprise at least one double bond. Specific examples include groups derived from pyran, morpholine, tetrahydrofuran, dihydrofuran, tetrahydropyran, dihydropyran, 1,3-dioxane, piperazine, piperidine, thiomorpholine, and such.

The aryl group described herein means a monocyclic or polycyclic hydrocarbon group that has aromaticity. Specific examples include groups derived from benzene, naphthalene, anthracene, and fluorene.

The heteroaryl group described herein means a four- to six-membered monocyclic or seven- to ten-membered bicyclic group (preferably a monocyclic group) which has aromaticity, and comprises one to four (preferably one or two) heteroatoms that are individually selected from nitrogen, sulfur, and oxygen atoms as ring members. Specific examples include groups derived from furan, thiophene, pyrrole, diazole, pyridine, thiazole, imidazole, pyrimidine, indole, quinoline, oxazole, isoxazole, pyrazine, triazole, thiadiazole, tetrazole, and pyrazole.

The aralkyl group described herein means the above-mentioned linear or branched alkyl group substituted with the above-mentioned aryl group, and specific examples include a benzyl group and a phenethyl group.

The heteroarylalkyl group described herein means the above-mentioned linear or branched alkyl group substituted with the above-mentioned heteroaryl group.

The acyl group described herein means the above-mentioned linear or branched alkyl group, aryl group, heteroaryl group, or heterocyclic group that is bonded via a carbonyl group.

The phrase "optionally substituted" described herein, unless particularly defined herein, means that a group may be substituted with a group such as a linear or branched alkyl group, linear or branched alkoxy group, linear or branched alkenyl group, linear or branched alkenyloxy group, linear or branched alkynyl group, linear or branched alkynyloxy group, cycloalkyl group, cycloalkyloxy group, cyano group, nitro group, trifluoromethyl group, trifluoromethoxy group, halogen atom, aryl group, aryloxy group, heteroaryl group, heteroaryloxy group, aralkyl group, aralkyloxy group, amino group (which is optionally mono- or di-substituted with a linear or branched alkyl group), acyl group, linear or branched alkylsulfonyl group, carbamoyl group, linear or branched alkylthio group, carboxyl group, linear or branched alkylcarbonyl group, formyl group, and aminosulfonyl group. The aryl and heteroaryl moieties included in these substituent groups may be further mono-, di-, or tri-substituted with a halogen atom, linear or branched alkyl group, linear or branched alkoxy group, linear or branched alkenyl group, linear or branched alkenyloxy group, linear or branched alkynyl group, linear or branched alkynyloxy group, cycloalkyl group, cycloalkyloxy group, cyano group, nitro group, trifluoromethyl group, trifluoromethoxy group, halogen atom, aryl group, aryloxy group, heteroaryl group, aralkyl group, aralkyloxy group, amino group that is optionally mono- or di-substituted with a linear or branched alkyl group, acyl group, linear or branched alkylsulfonyl group, linear or branched alkoxy group, carbamoyl group, linear or branched alkylthio group, carboxyl group, linear or branched alkylcarbonyl group, formyl group, aminosulfonyl group, and such.

Furthermore, pharmaceutically acceptable salts of the compounds represented by formula (I) can be produced by contacting the compounds with an acid or base that can be used in producing pharmaceuticals. The salts are not particularly limited as long as they are pharmaceutically acceptable, and include, for example, salts with mineral acids such as hydrochloric acid, sulfuric acid, nitric acid, phosphoric acid, and hydrobromic acid; salts with organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid; and salts with alkali metals and alkali earth metals such as sodium, potassium, and calcium.

Furthermore, the salts include hydrates and solvates that may be formed by the compounds. When a compound represented by formula (I) is obtained in a free form, it can be converted into salts or their hydrates or solvates that may be formed by the compound, according to conventional methods.

The compounds represented by formula (I) of the present invention can be synthesized by methods described in WO2004/071503, WO2005/005372, and WO2006/016657.

Preferred examples of the compounds represented by formula (I) of the present invention include the following compounds:

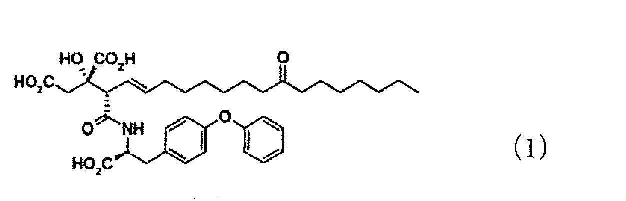

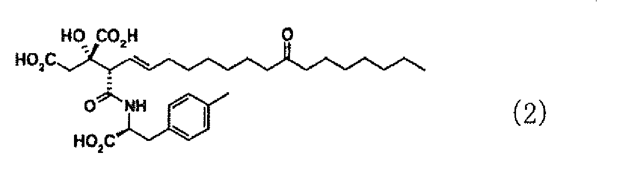

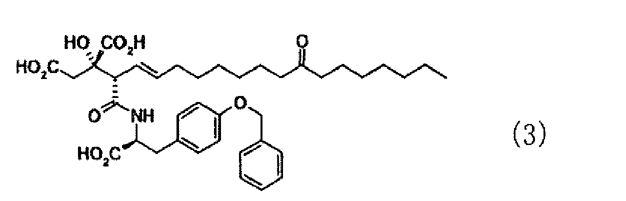

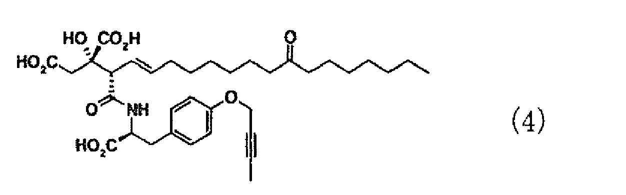

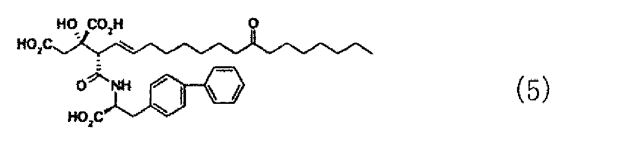

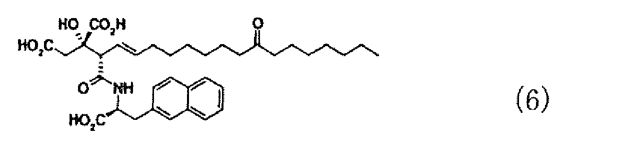

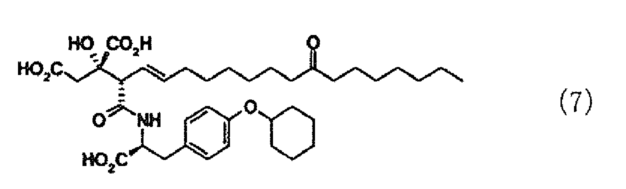

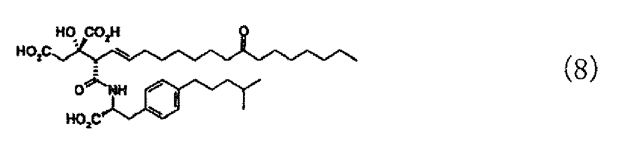

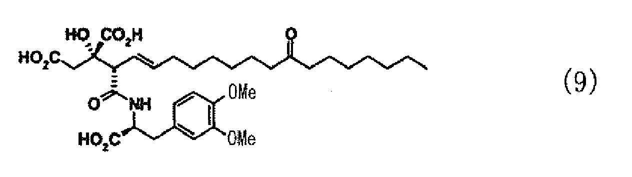

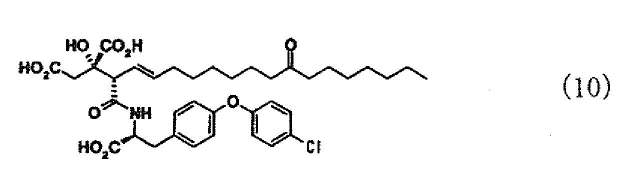

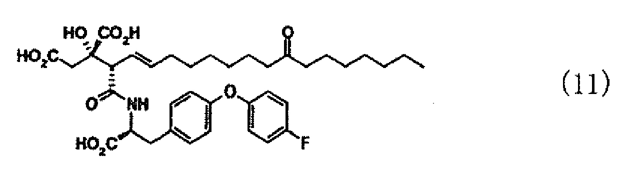

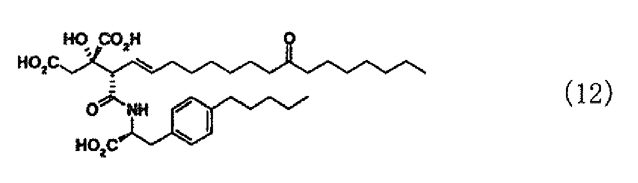

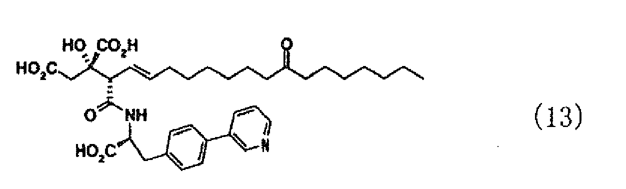

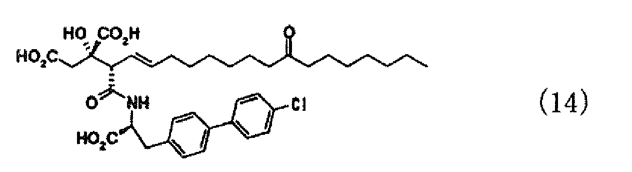

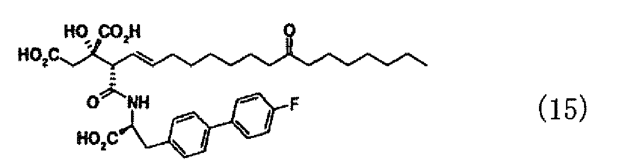

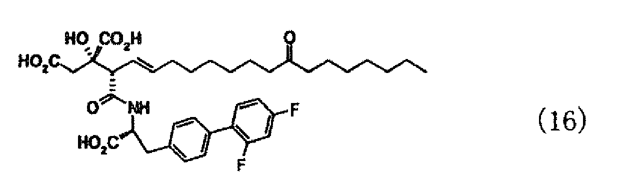

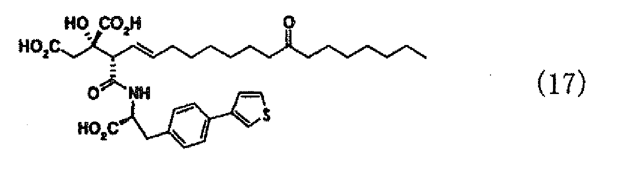

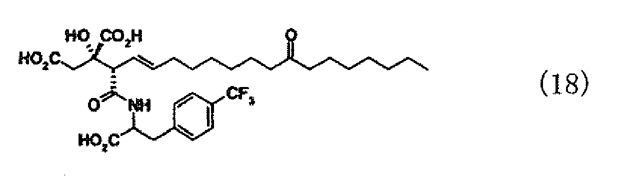

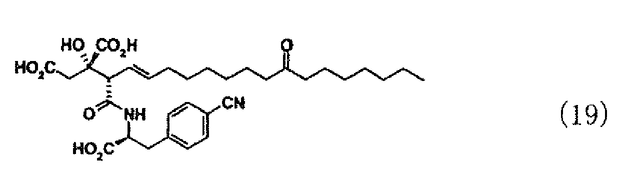

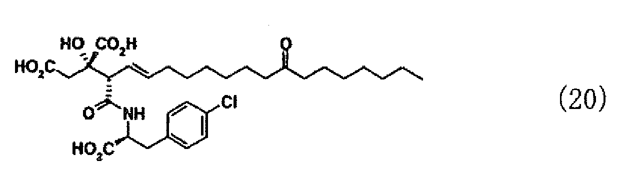

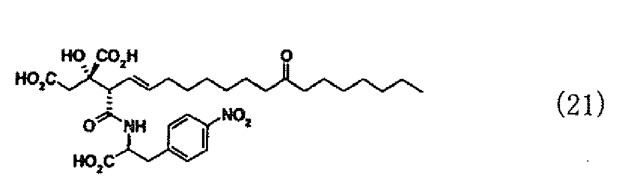

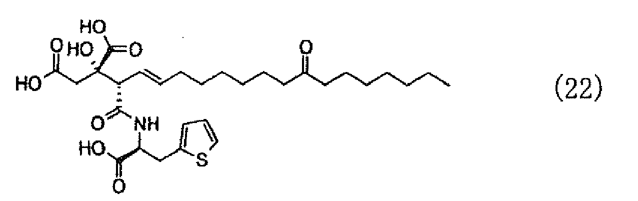

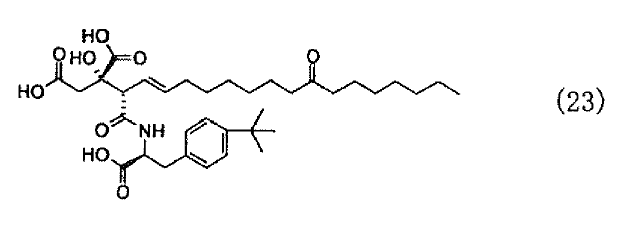

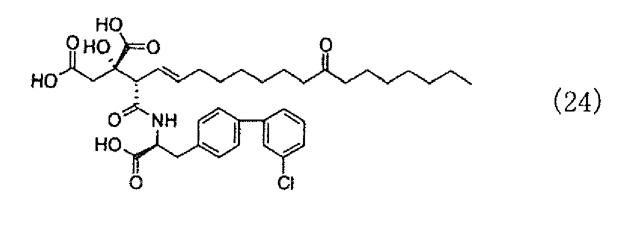

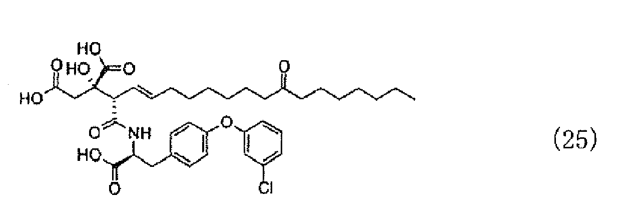

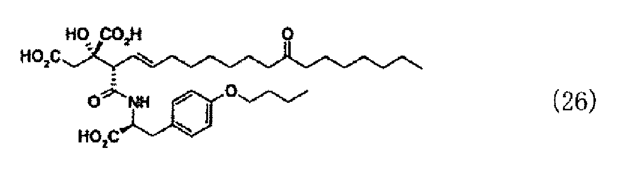

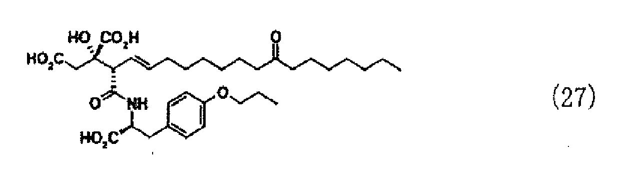

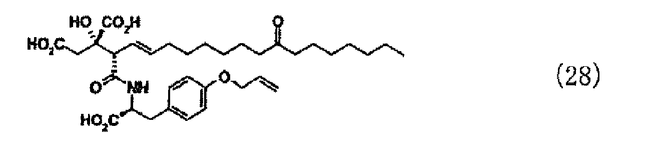

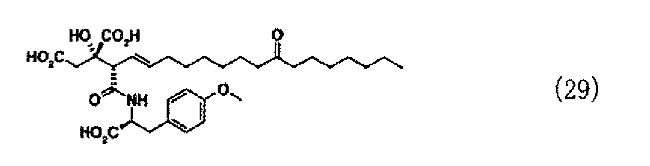

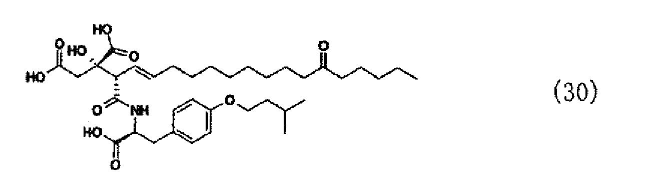

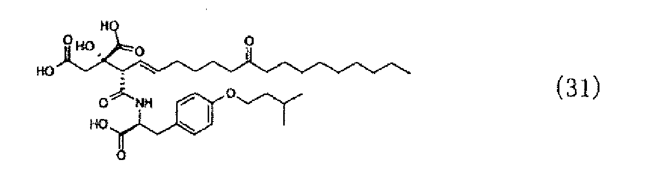

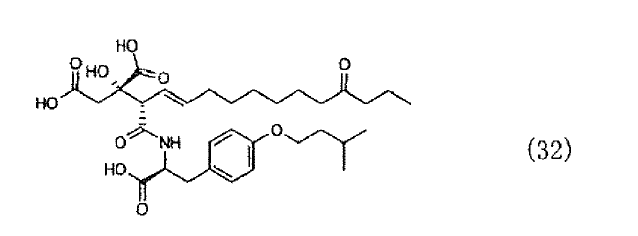

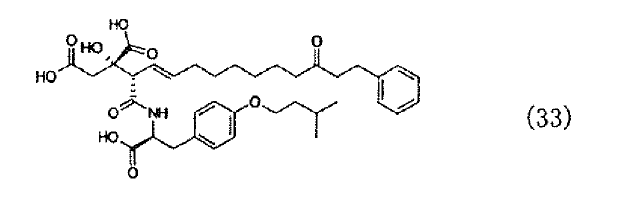

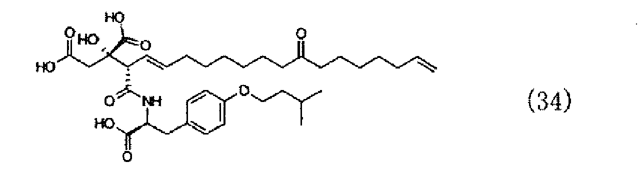

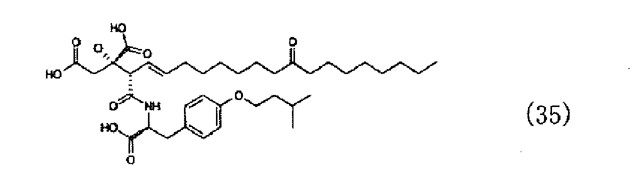

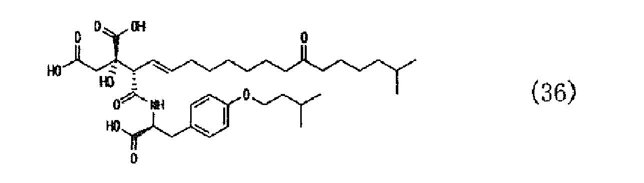

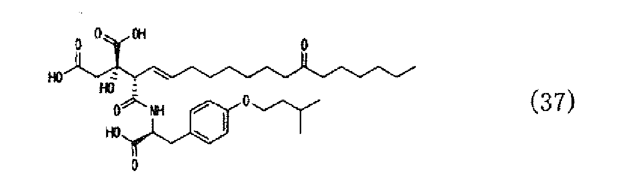

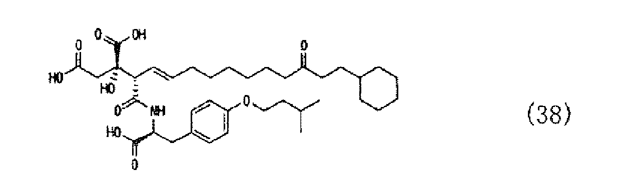

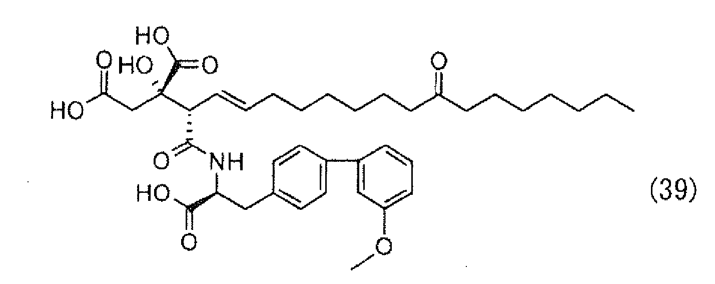

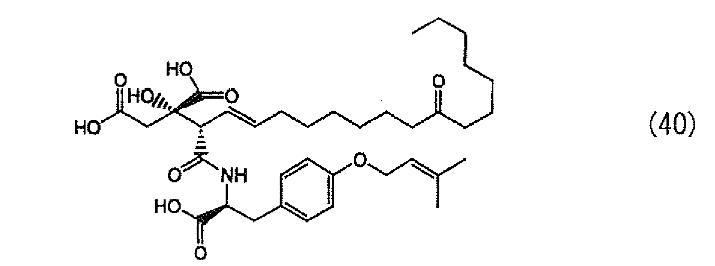

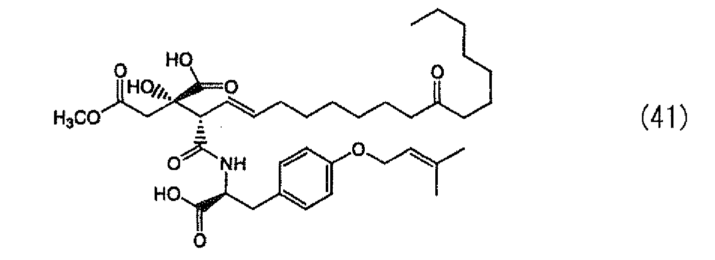

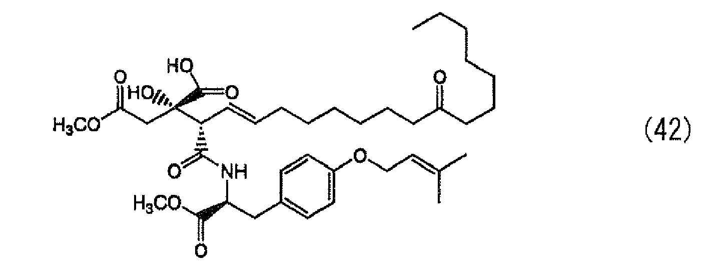

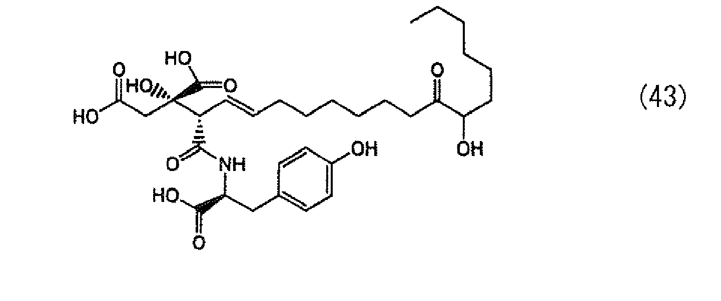

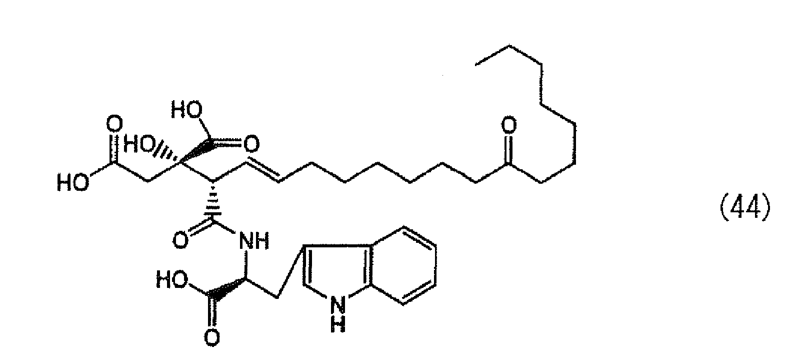

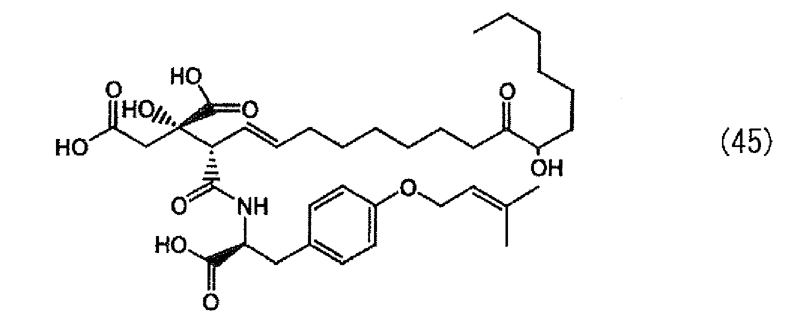

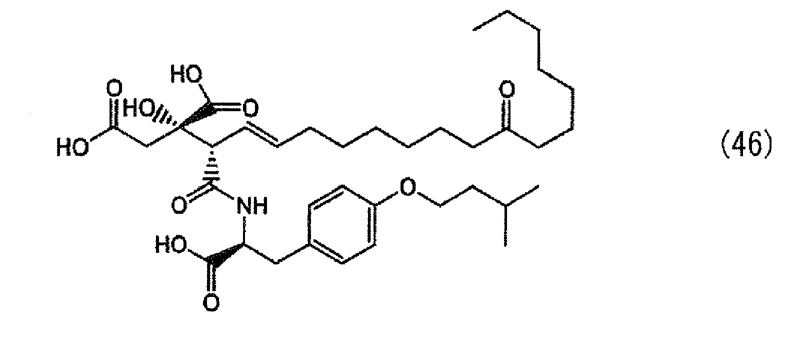

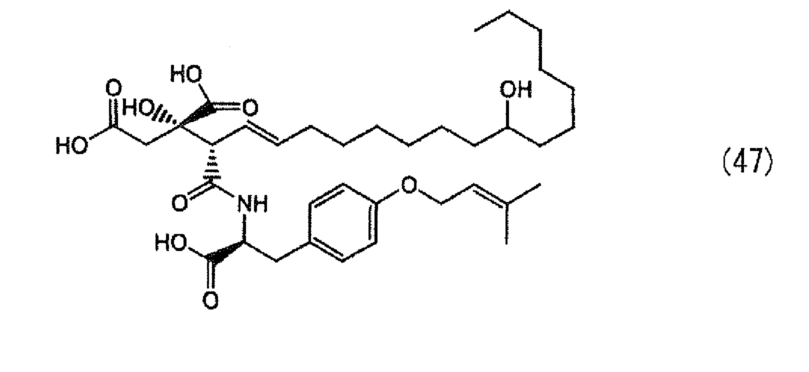

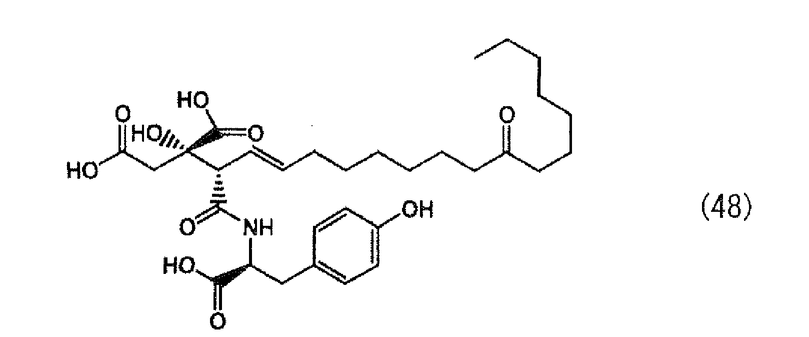

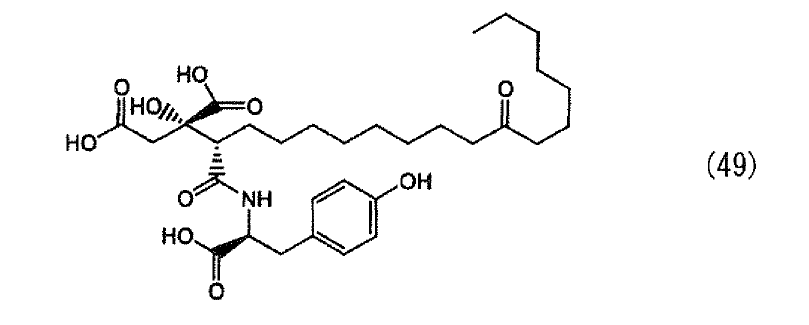

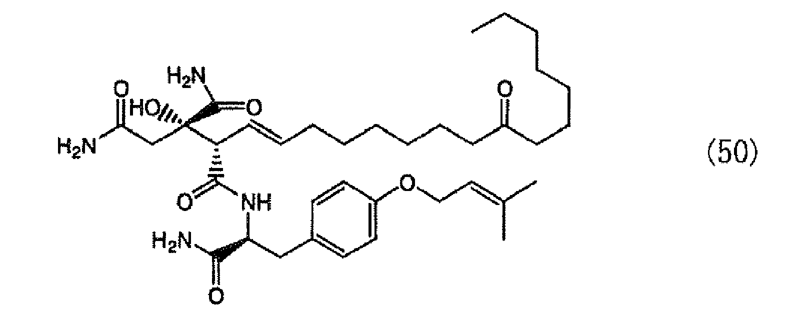

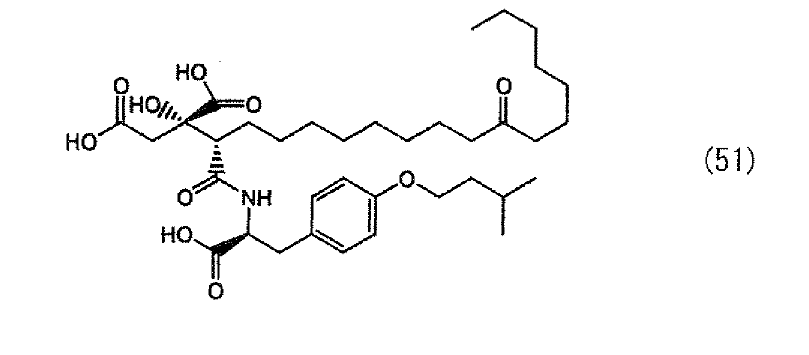

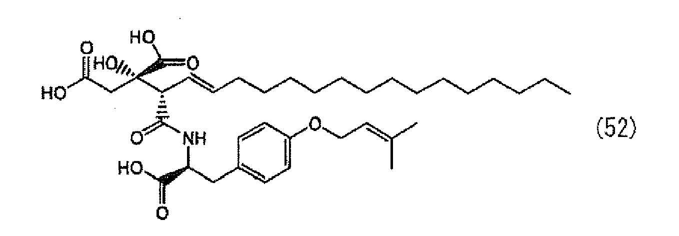

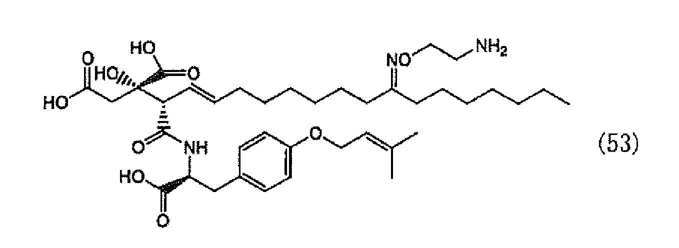

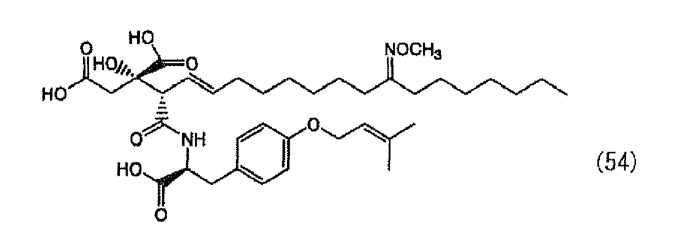

More preferred examples of the compounds represented by formula (I) of the present invention include the compounds represented by formulae (II) to (XII).

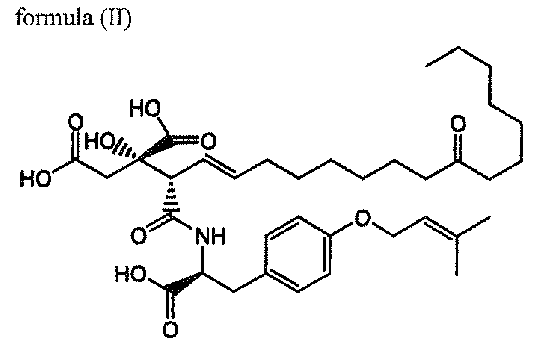

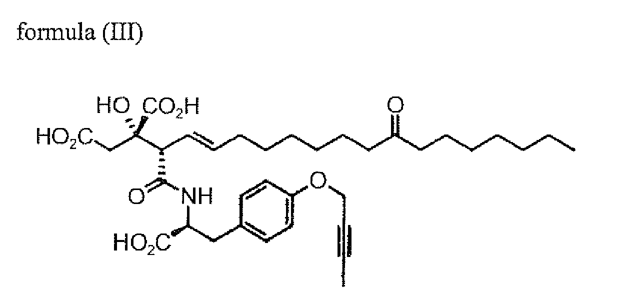

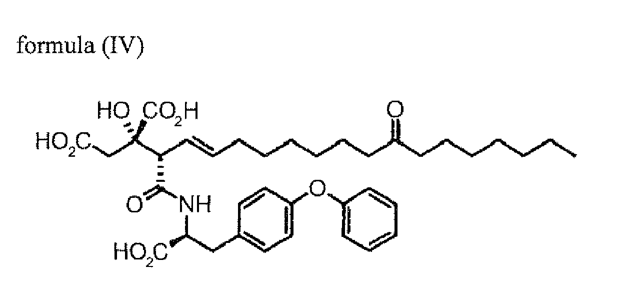

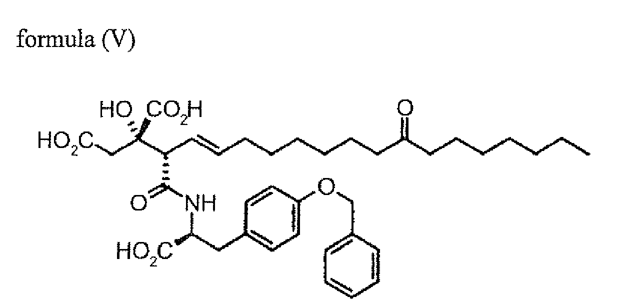

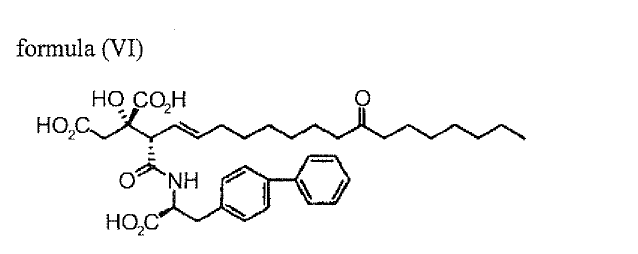

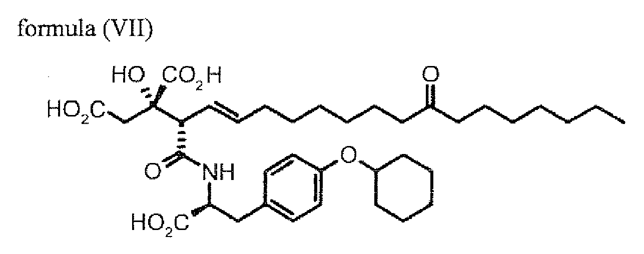

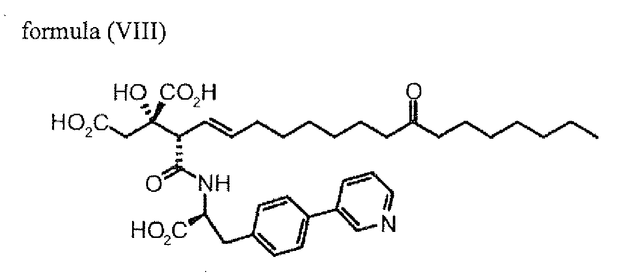

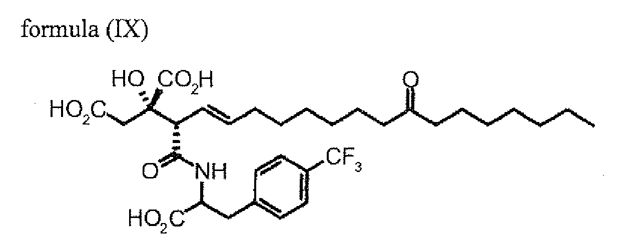

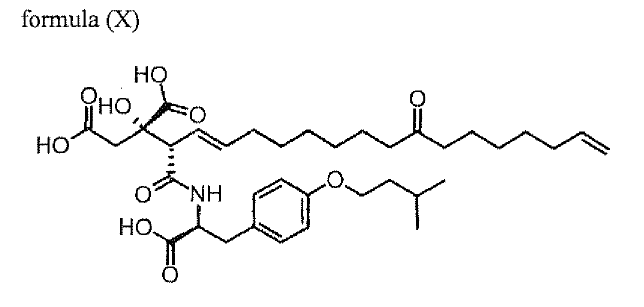

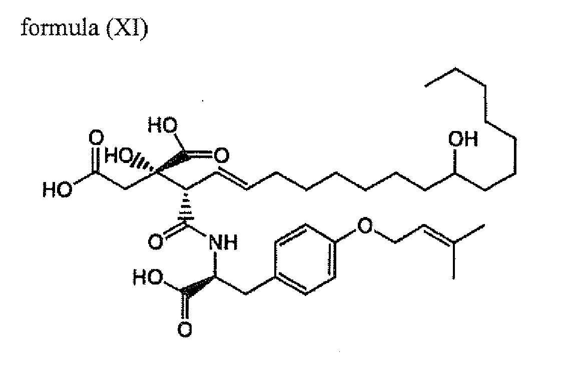

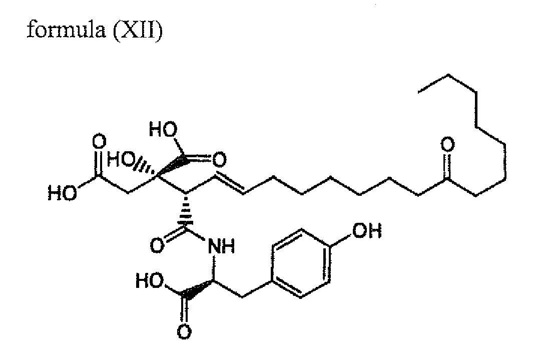

Herein, the term "therapy" means that HBV is eliminated or reduced, further propagation of HBV is suppressed, or symptoms caused by HBV infection are relieved by administering the agents of the present invention to subjects. Such therapy includes, for example, exclusion of HBV, subsidence of hepatitis, and prevention or reduction of progression from hepatitis to cirrhosis, hepatic fibrosis, and liver cancer. Meanwhile, the term "prevention" means that HBV infection is prevented or HBV propagation is suppressed by administration to subjects before they are infected with HBV.

Herein, the term "HBV" refers to all viruses capable of causing hepatitis B. To date, genotypes A to H are known for HBV. Targets for therapy and prevention using the agents of the present invention are not particularly limited, and include all genotypes.

Herein, the term "HBV infection" refers to all symptoms caused by infection of the above-described HBV in living organisms including humans. Specifically, such symptoms include, for example, hepatitis B, cirrhosis, hepatic fibrosis, and liver cancer. Hepatitis B is further classified into chronic hepatitis, acute hepatitis, and fulminant hepatitis. Liver cancer includes, for example, hepatocellular carcinoma. Whether a living organism has an HBV infection can be evaluated, for example, by detecting HBs or HBe antigen in blood, or by measuring the level of HBV-DNA or HBV DNA polymerase in blood, or using a combination thereof.

The compounds of the present invention can be used as pharmaceutical agents directly or in the form of pharmaceutically acceptable salts. The above-mentioned salts are not particularly limited, so long as they are pharmaceutically acceptable, and examples include salts formed with mineral acids such as hydrochloric acid, sulfuric acid, nitric acid, phosphoric acid, and hydrobromic acid; salts formed with organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid; and salts formed with alkali metals or alkaline earth metals such as sodium, potassium, and calcium.

The amount of an active ingredient compound comprised in the above-mentioned pharmaceutical preparation is not particularly limited and can be appropriately selected in a wide range; however, examples are 0.1% to 99.5% by weight, or preferably 0.5% to 90% by weight.

A compound of the present invention can be formulated as the base according to conventional methods using known adjuvants that may be used ordinarily in the art of pharmaceutical preparation, such as excipients, binders, disintegrators, lubricants, flavoring agents, solubilizing adjuvants, suspending agents, and coating agents. When shaping into the form of tablets, a wide variety of substances conventionally known as carriers in the art can be used, and examples include excipients such as lactose, sucrose, sodium chloride, glucose, urea, starch, calcium carbonate, kaolin, crystalline cellulose, and silicic acid; binders such as water, ethanol, propanol, simple syrup, glucose solution, starch solution, gelatin solution, carboxymethylcellulose, shellac, methylcellulose, potassium phosphate, and polyvinylpyrrolidone; disintegrators such as dried starch, sodium alginate, agar powder, laminaran powder, sodium bicarbonate, calcium carbonate, polyoxyethylene sorbitan fatty acid esters, sodium lauryl sulfate, stearic acid monoglyceride, starch, and lactose; disintegration inhibitors such as sucrose, stearic acid, cacao butter, and hydrogenated oil; absorbefacients such as quaternary ammonium salts and sodium lauryl sulfate; moisturizers such as glycerin and starch; adsorbents such as starch, lactose, kaolin, bentonite, and colloidal silicic acid; and lubricants such as purified talc, stearate, boric acid powder, and polyethylene glycol.

Tablets can be prepared, as necessary, as ordinary coated tablets, such as sugar-coated tablets, gelatin-coated tablets, enteric-coated tablets, and film-coated tablets, or as double layered tablets or multilayered tablets. When shaping into the form of pills, a wide variety of substances conventionally known as carriers in the art can be used, and examples include excipients such as glucose, lactose, cacao butter, starch, hardened vegetable oil, kaolin, and talc; binders such as gum arabic powder, tragacanth powder, gelatin, and ethanol; and disintegrators such as laminaran agar. When shaping into the form of suppositories, a wide variety of substances conventionally known as carriers in this field can be used, and examples include polyethylene glycol, cacao butter, higher alcohols, esters of higher alcohols, gelatin, and semi-synthetic glycerides. When preparing injections, solutions and suspensions are sterilized and are preferably isotonic with blood, and when making these solutions, emulsions, and suspension forms, any substances commonly used as diluents in the field can be used, such as water, ethanol, propylene glycol, ethoxylated isostearyl alcohol, polyoxylated isostearyl alcohol, and polyoxyethylene sorbitan fatty acid esters. In these instances, adequate amounts of sodium chloride, glucose, or glycerin can be comprised in the pharmaceutical preparations to prepare isotonic solutions, and ordinary solubilizing adjuvants, buffers, analgesic agents, and such may also be added. The pharmaceutical preparations may further comprise, as necessary, coloring agents, preservatives, flavors, flavoring agents, and sweeteners, as well as other pharmaceutical agents.

The above-mentioned pharmaceutical compositions are preferably administered in unit dosage forms, and can be administered orally, interstitially (subcutaneously, intramuscularly, intravenously, and such), locally (percutaneously), or transrectally. The pharmaceutical compositions are obviously administered in dosage forms suited to these administration methods.

When administering the compounds of the present invention or pharmaceutically acceptable salts thereof as pharmaceutical agents, the doses of the antiviral agents are preferably adjusted after considering the patient's conditions such as age and weight, the administration route, and the property and degree of the disease; however, for humans, the daily dose of the active ingredient of the present invention for adults is ordinarily within the range of 0.1 mg to 2,000 mg. While doses lower than the above-mentioned range may be sufficient in some cases, doses higher than this range may be required in other cases. When a high dose is used, the daily dosage is preferably administered in several divided doses.

The above-mentioned oral administration can be carried out using solid, powdered, or liquid dosage units, such as powders, powdered drugs, tablets, sugar-coated agents, capsules, drops, sublingual tablets, and other dosage forms.

The above-mentioned interstitial administration can be carried out, for example, using liquid unit dosage forms for subcutaneous, intramuscular, or intravenous injections, such as solutions and suspensions. These are produced by suspending or dissolving a certain amount of a compound of the present invention or a pharmaceutically acceptable salt thereof, in a non-toxic liquid carrier suitable for purposes of injection, such as an aqueous or oily medium, and then sterilizing this suspension or solution.

The above-mentioned local administration (percutaneous administration and such) can be carried out using external preparation forms such as solutions, creams, powders, pastes, gels, and ointments. These are produced by combining a certain amount of a compound of the present invention or a pharmaceutically acceptable salt thereof, with one or more of a flavor, coloring agent, filler, surfactant, moisturizer, emollient, gelling agent, carrier, preservative, and stabilizer suited to the aim of the external preparation.

The above-mentioned transrectal administration can be carried out using suppositories and the like, prepared by mixing a certain amount of a compound of the present invention or a pharmaceutically acceptable salt thereof into a low-melting solid comprising, for example, higher esters such as myristyl palmitate ester, polyethylene glycol, cacao butter, or a mixture thereof.

The above-mentioned administrations can be carried out using liquid unit dosage forms for subcutaneous, intramuscular, or intravenous injections, such as solutions or suspensions. They are produced by suspending or dissolving a certain amount of a compound of the present invention or a pharmaceutically acceptable salt thereof, in a non-toxic liquid carrier appropriate to the purpose of the injection, such as an aqueous or oily medium, and then sterilizing this suspension or solution.

The anti-HBV agents of the present invention may be used alone or in combination with other anti-HBV agents.

Thus, the present invention also provides pharmaceutical compositions for treating or preventing HBV infection, which comprise a compound represented by formula (I) above in combination with one or more other anti-HBV agents. The pharmaceutical compositions may be those which comprise a compound represented by formula (I) in combination with other anti-HBV agents which are administered simultaneously, separately, or in succession for treating or preventing HBV infection. The pharmaceutical compositions of the present invention may be combination agents that are prepared by formulating a compound represented by formula (I) in combination with other anti-HBV agents into the same pharmaceutical composition, or combination agents in which a compound represented by formula (I) and other anti-HBV agents are separately formulated into different pharmaceutical compositions. The ratio between a compound represented by formula (I) and interferons in such combination agents can be a predetermined ratio or be varied to any ratio at the time of administration depending on symptom severity, clinicians' decision, and such. Alternatively, the pharmaceutical compositions of the present invention may be kits prepared by combining a pharmaceutical composition including a compound represented by formula (I) with a pharmaceutical composition(s) including other anti-HBV agents.

In the above-described "pharmaceutical compositions for treating or preventing HBV infection which comprise the compound represented by formula (I) in combination with other anti-HBV agents", when the compound represented by formula (I) and other anti-HBV agents are each formulated separately in different pharmaceutical compositions, the dosage forms of the two or more preparations may be identical or different. For example, one or more preparations may be parenteral preparations, injections, drops, or intravenous drips.

The present invention also relates to pharmaceutical compositions for treating or preventing HBV infection, which comprise as an active ingredient a compound represented by formula (I), and which are used in combination with other anti-HBV agents. When the pharmaceutical compositions are used in combination with other anti-HBV agents, they may be administered simultaneously along with other anti-HBV agents, or administered before or after administration of other anti-HBV agents.

Herein, "other anti-HBV agents" include interferons (IFN) and nucleic acid analogs (Lamivudine [LMV], Adefovir [ADV], and Entecavir [ETV]), preferably interferons and Entecavir, and more preferably PEGylated interferon and Entecavir.

In the present invention, "nucleic acid analog" is a generic term for a group of compounds having a nucleic acid (adenine, guanine, cytosine, thymine, or uracil) moiety in their molecules and having the activity of inhibiting DNA synthesis of HBV.

Lamivudine can be used by synthesizing, for example, by the method described in EP382526. Adefovir can be used by synthesizing, for example, by the method described in EP206459. Adefovir may be used in a form of pro-drug, such as a di(pivaloylmethyl)cster, i.e., Adefovir Dipivoxil. Such pro-drugs can be used by synthesizing, for example, by the method described in EP481214. Entecavir can be used by synthesizing, for example, by the method described in EP481754. Furthermore, nucleic acid analogs available as a reagent or the like on the market can be purchased and used. Alternatively, nucleic acid analogs approved as drugs can be obtained as formulations and used. For example, Lamivudine and Adefovir Pivoxil are manufactured and sold by Glaxo Smith Kline K.K., while Entecavir is manufactured and sold as a hydrate by Bristol-Myers.

Using in combination with interferons or nucleic acid analogs, the compound represented by formula (I) can provide therapeutic or preventive methods that are also elective against HBV that is resistant to a nucleic acid analog. Specifically, pharmaceutical compositions for treating or preventing HBV infection, which comprise a compound represented by formula (I) above in combination with interferons or nucleic acid analogs are also effective against HBV that is resistant to nucleic acid analogs.

Alternatively, a compound represented by formula (I) alone can be formulated as pharmaceutical compositions for treating or preventing HBV infection caused by HBVs that is resistant to nucleic acid analogs.

In such cases, HBV may be resistant to one, two or more nucleic acid analogs. For example, HBV may be resistant to either or both of Lamivudine and Entecavir.

Herein, the phrase "HBV that is resistant to nucleic acid analogs" refers to a HBV in which the amino acid sequences produced from the viral DNA have one or more mutations (substitutions, additions, and/or deletions) as compared to the amino acid sequences produced from the DNA of wild-type HBV strain of the same genotype. By causing mutations, HBV can avoid the pressure of exposure to nucleic acid analogs and acquire resistance to the nucleic acid analogs. Nucleic acid analogs to which HBV show resistance can vary depending on the mode of mutation, such as the positions and number of substitutions, additions, and/or deletions in the amino acid sequence. In the present invention, the "HBV that is resistant to nucleic acid analogs" includes all HBV having one or more mutations, regardless of such differences.

In the present invention, "interferon" collectively refers to proteins or glycoproteins that have an antiviral action and are induced from animal cells by viruses, double stranded RNA, lectin, and such. In addition to the antiviral action, interferons have a cell growth-suppressing action and an immunoregulatory action. They are categorized into several types according to the cells producing them, the binding ability to specific receptors, and biological and physicochemical characteristics. The major types are α, β, and γ, and other types that are known to exist are IFNω, and IFNτ. Furthermore, 20 or more subtypes of interferon α are known to exist. At present, not only naturally-derived formulations but also various genetically recombinant type formulations, such as PEG-interferons and consensus interferons have been developed and are commercially available.

The interferons of the present invention may be of any type described above; however, interferons α and γ are preferred. Furthermore, the interferons of the present invention may be a natural type, genetic recombinant type which is artificially mutated, naturally-occurring mutant, fusion protein, a fragment thereof, or such, as long as it enhances the ability to suppress the HBV proliferation when used in combination with the compound represented by formula (I). Furthermore, the interferons of the present invention may be PEG(polyethylene glycol)ylated. Interferons can be pegylated by methods known to those skilled in the art (Japanese Patent No. 2980569). PEGylated interferons include, for example, Peginterferon α-2a (Pegasys^{™}) and Peginterferon α-2b (Peglntron^{™}). PEG-IFN used in Examples 2 to 5 herein refers to Peginterferon α-2a. Furthermore, interferons of the present invention may be an interferon that is linked to albumin. Such albumin-linked interferons include, for example, Albinterferon α-2b (Albuferon^{™})_{.}

The interferons according to the present invention are not particularly limited in terms of their origin. For example, the interferons can be derived from humans, chimpanzees, orangutans, dogs, horses, sheep, goats, donkeys, pigs, cats, mice, guinea pigs, rats, rabbits, or such; however, the origin is not limited thereto, and the interferons can also be derived from other mammals. Preferably, the interferons are derived from humans.

The amino acid sequences of human interferons α and γ are known. For example, the amino acid sequence of GenBank: NM_0240013 can be used for interferon α, and the amino acid sequence of GenBank: NM_000619 can be used for interferon γ.

Alternatively, it is also possible to further combine interferons with pharmaceutical compositions for treating or preventing HBV infection, which comprises a compound represented by formula (I) above in combination with nucleic acid analogs.

All prior art documents cited herein are incorporated by reference herein.

### Examples

Hereinbelow, the present invention will be specifically described with reference to Examples.

### [Example 1] Anti-HBV effect of compound represented by formula (III)

The present inventors assessed the anti-HBV effect of the compound represented by formula (III) on Huh-7 cells infected with HBV (genotype C_AT; wild-type strain).

On the day before HBV infection, about 100,000 Huh-7 cells were plated in 12-well plates (FALCON, 353043) using culture medium DMEM (SIGMA, D6429).

On the day of infection, 50 µl of a homogenous mixture of 0.5 µg of HBV-DNA (described in "Sugiyama M, et al. HEPATOLOGY, Vol. 44: 915-924, 2006"), 1.5 µl of FUGENE6 (Roche Diagnostics; 11 814 443 001), 5 µl of SEAP, and 43 µl of OPTIMEM (Invitrogen; 31985) was added to the Huh-7 cells in each well. Then, the compound represented by formula (III) was added at a final concentration of 0, 3, 10, or 30 µM.

The cells were cultured at 37°C under 5% CO₂ for three days, and then culture supernatant was collected from each well. The supernatant was treated with deoxyribonuclease (DNase) to remove free DNA. Specifically, mixtures of 50 µl of the culture supernatant, 10 µl of 10x DNase buffer (Promega, M6101), 20 µl of DNase (Promega, M6101), and 20 µl of sterile water were incubated at 37°C for 30 minutes, and then 20 µl of a reaction-termination solution and 80 µl of sterile water were added thereto. The resulting mixtures were incubated at 65°C for ten minutes.

HBV was quantified by measuring HBV-DNA using real-time PCR (Device used: Applied Biosystems, 4318157). First, using QIAGEN QIAamp DNA Blood Mini Kit (QIAGEN; 51106), 50 µl of DNA was extracted from 200 µl of the supernatants eluted after DNase treatment. Five µl of the DNA solutions were each mixed with 5 µl of sterile water, 12.5 µl of Master Mixture, 0.5 µl of TaqManProbe HBVSP2 solution, 0.5 µl of Primer F (HBVS190F) solution, and 0.5 µl of Primer R (HBVS703R) solution (HBVS190F (190-207) has the sequence of: GCTCGTGTTACAGGCGGG (SEQ ID NO: 1); HBVS703R (684-709) has the sequence of: GAACCACTGAACAAATGGCACTAGTA (SEQ ID NO: 2); and HBVSP2 has the sequence of: FAM-atgttgcccgtttgtcctctaattccag-TAMRA (SEQ ID NO: 3)). After PCR (condition: 95°C for 10 minutes; followed by 35 cycles of [95°C for 30 seconds, 60°C for 30 seconds, and 72°C for 45 seconds]) was conducted, the samples were stored at 4°C. HBV-DNA copy numbers were determined from the obtained fluorescent signals.

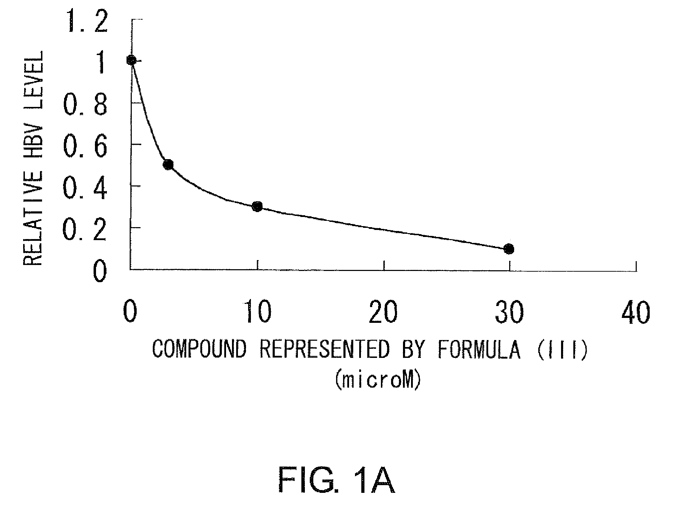

The result showed that 50%-inhibition concentration (IC₅₀) of the compound represented by formula (III) was about 3 µM (Fig. 1A).

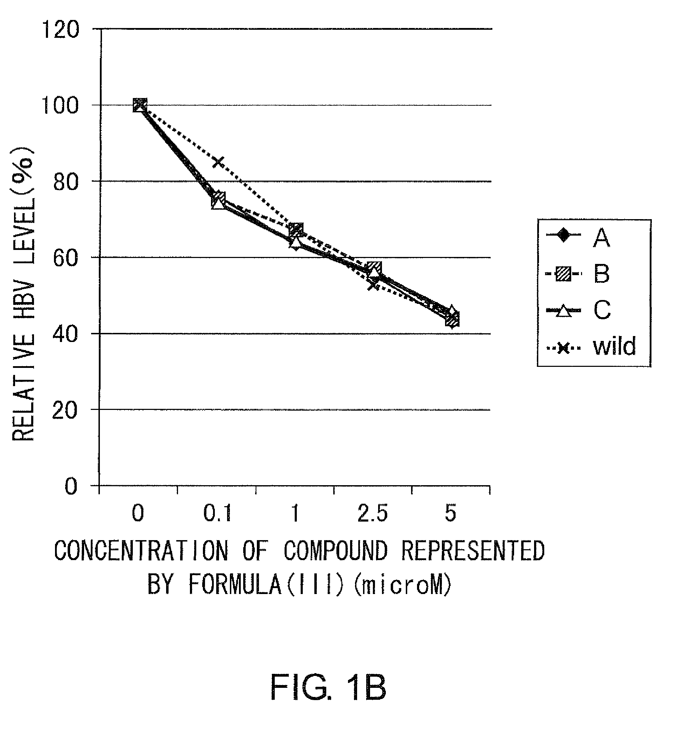

Furthermore, the effect of suppressing HBV propagation was assessed by the same method using Lamivudine- and Entecavir-resistant strains (A, L180M+M204V; B, L180M+M204V+T184L; C, M204V(YVDD)) as well as wild-type HBV strain (Wild). The result showed that the compound represented by formula (III) exerted a comparable HBV propagation-suppressing effect whether or not HBV was resistant to the agents (Fig. 1B).

### [Example 2] Anti-HBV effect of the compound represented by formula (III) used in combination with PEG-IFN on HBV-infected chimeric mice (1).

The inhibitory activity of the compound represented by formula (III) was assessed using chimeric mice (purchased from PhoenixBio Co., Ltd.) having human liver infected with wild-type HBV strain (genotype C_AT; wild-type strain).

Specifically, the compound represented by formula (III) and/or PEG-IFN (Chugai Pharmaceutical Co.) were administered by intravenous or subcutaneous injection to mice infected with genotype C HBV (C_JPNAT; reference: Sugiyama) according to Table 1. Then, blood was collected from the mice.

**[Table 1]**

| SCHDULE OF ADMIINISTRATION TO CHIMERIC MICE INFECTED WITH GENOTYPE C HBV | | | | | | | | | | | | | | | | |
|---|---|---|---|---|---|---|---|---|---|---|---|---|---|---|---|---|
| Day | -1 | 0 | 1 | 2 | 3 | 4 | 5 | 6 | 7 | 8 | 9 | 10 | 11 | 12 | 13 | 14 |
| BLOOD SAMPLING | ○ | | | ○ | | | ○ | | | ○ | | | ○ | | | ○ |
| PEG-IFN | | 30 | | | 30 | | | 30 | | | 30 | | | 30 | | |
| COMPOUND REPRESENTED BY FORUMULA (III)+PEG-IFN | | 10+30 | 10 | 10 | 10+30 | 10 | 10 | 10+30 | 10 | 10 | 10+30 | 10 | 10 | 10+30 | 10 | 10 |

In Table 1, circle represents the timing of blood collection; 30 indicates administration of PEG-TFN at 30 µg/kg; 10+30 indicates administration of the compound represented by formula (III) at 10 mg/kg in combination with PEG-IFN at 30 µg/kg.

Next, serum HBV was purified by the same method as described in Example 1, and HBV was quantified by measuring HBV-DNA using real-time PCR method. The sequences of primers HBSF2: 5'-CTT CAT CCT GCT GCT ATG CCT-3'[nt 406-426] (SEQ ID NO: 4) and HBSR2: 5'-AAA GCC CAG GAT GAT GGG AT-3'[nt 646-627] (SEQ ID NO: 5), and the sequence of TaqMan probe HBSP2 (nt 461-488) (5'-ATG TTG CCC GTT TGT CCT CTAATT CCA-3' (SEQ ID NO: 6)) were the same as those described in the reference (Abe A, Inoue K, Tanaka T, Kato J, Kajiyama N, Kawaguchi R, Tanaka S, et al. Quantitation of hepatitis B virus genomic DNA by real-time detection PCR. J Clin Microbiol (1999) 37: 2899-2903).

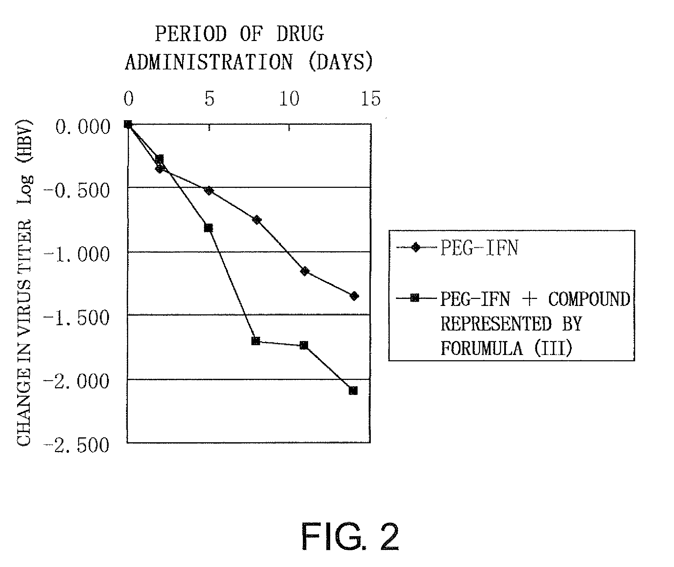

The result showed that the serum level of HBV DNA was decreased by about 1.4 Log after 14 days in the PEG-IFN-treated group. Meanwhile, the HBV level was decreased by about 2 Log in the group treated with PEG-IFN in combination with the compound represented by formula (III) (Fig. 2). This finding demonstrates that the anti-HBV effect of PEG-TFN is enhanced by using it in combination with the compound represented by formula (III) above.

### [Example 3] Anti-HBV effect of the compound represented by formula (III) used in combination with PEG-IFN on HBV-infected chimeric mice (2)

The same test as described in Example 2 was carried out using chimeric mice (purchased from PhoenixBio Co., Ltd.) having human liver infected with HBV genotype A (wild-type strain).

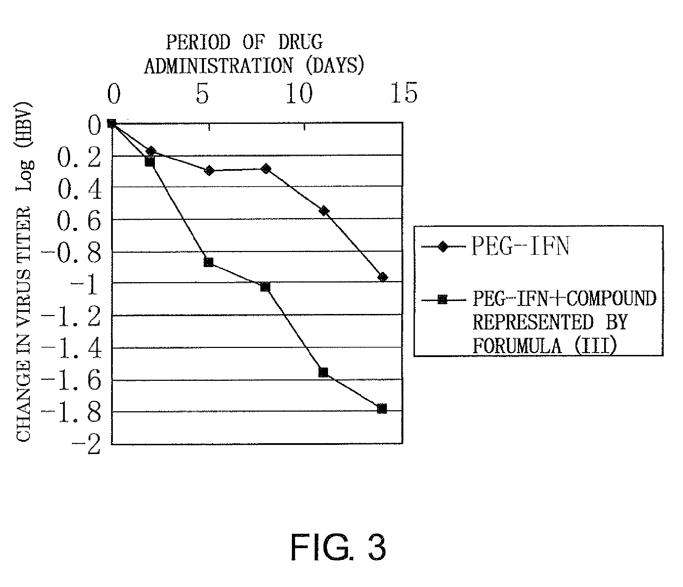

The result showed that the serum level of HBV DNA was decreased by about 1 Log after 14 days in the PEG-IFN-treated group. Meanwhile, the HBV level was decreased by about 1.8 Log in the group treated with PEG-IFN in combination with the compound represented by formula (III) (Fig. 3). This finding demonstrates that the anti-HBV effect of PEG-IFN against genotype A was also enhanced when PEG-IFN was used in combination with the compound represented by formula (III) above.

### [Example 4] Anti-HBV effect of the compound represented by formula (III) used in combination with Entecavir on HBV-infected chimeric mice (1), and anti-HBV effect of the compound represented by formula (III) used in combination with PEG-IFN on HBV-infected chimeric mice (3)

The same test as described in Example 3 was carried out using chimeric mice (purchased from PhoenixBio Co., Ltd.) having human liver infected with HBV (genotype C_AT; wild-type strain). The compound represented by formula (III) was administered at 5 mg/kg (intravenous injection every day), while PEG-IFN was administered at 30 µg/kg (subcutaneous injection). Entecavir (ETV) was administered at 0.02 mg/kg (oral administration every day).

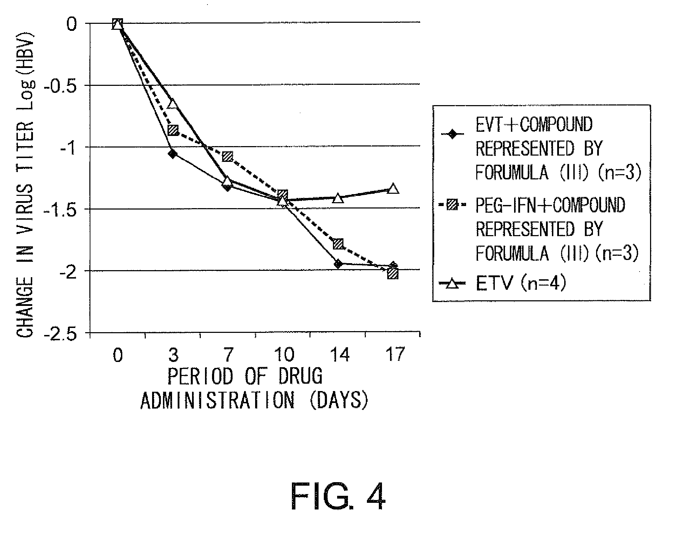

The result showed that, in the second week, the mean decrease of virus titer was 1.4 Log in the ETV-administered group (n = 4) and 2.0 Log in both group treated with ETV in combination with the compound represented by formula (III) (n = 3) and group treated with PEG-IFN in combination with the compound represented by formula (III) (n = 3) (Fig. 4). This result demonstrates that the anti-HBV activity of Entecavir is enhanced when Entecavir is used in combination with the compound represented by formula (III) above and the activity is comparable to the anti-HBV activity when PEG-IFN is used in combination with the compound represented by formula (III).

### [Example 5] Anti-HBV effect of the compound represented by formula (III) used in combination with Entecavir on HBV-infected chimeric mice (2), and anti-HBV effect of the compound represented by formula (III) used in combination with PEG-IFN on HBV-infected chimeric mice (4)

The same test as described in Example 3 was carried out using chimeric mice (purchased from PhoenixBio Co., Ltd.) having human liver infected with genotype C HBV (L180M+S202G+M204V; ETV-resistant strain). The compound represented by formula (III) was administered at 5 mg/kg (intravenous injection every day), while PEG-IFN was administered at 30 µg/kg (subcutaneous injection). Entecavir (ETV) was administered at 0.02 mg/kg (oral administration every day).

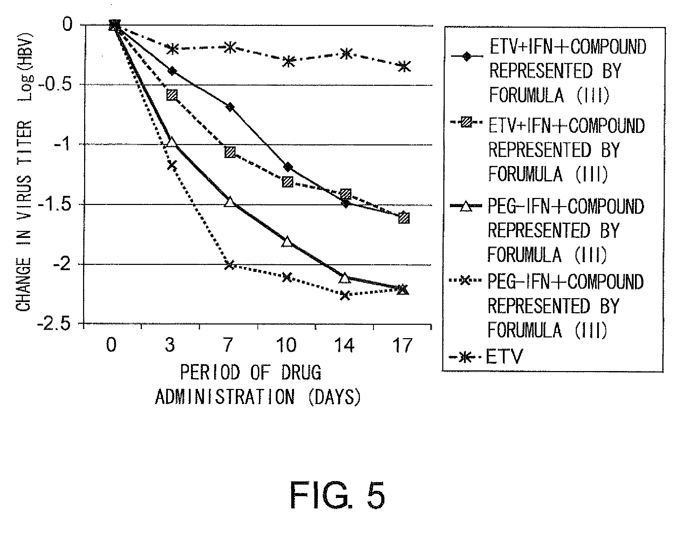

The result showed that the viral decrease was 0.3 Log in the ETV-resistant strain (n = 1). A maximum 1.6 Log decrease was observed in the group treated with ETV in combination with the compound represented by formula (III) (plotted at n = 1 x 2 instead of n = 2), while the decrease was 2.2 Log in the group treated PEG-IFN in combination with the compound represented by formula (III) (plotted at n = 1 x 2 instead of n = 2) (Fig. 5). This result demonstrates that the anti-HBV activity of Entecavir is also enhanced when Entecavir is used in combination with the compound represented by formula (III) above against HBV that is resistant to ETV. Furthermore, the enhancing effect was higher when PEG-IFN was used in combination with the compound represented by formula (III).

The Entecavir-resistant HBV strain used herein is also known to be resistant to other nucleic acid analogs such as Lamivudine.

### Industrial Applicability

The present invention provides novel pharmaceutical compositions for treating or preventing HBV infection. The pharmaceutical compositions of the present invention alone exert an anti-HBV effect and thus are useful as a therapeutic agent for HBV infection. The pharmaceutical compositions of the present invention are also useful in that they can be used as a novel HBV therapeutic agent having an effect against viruses resistant to drugs such as nucleic acid analogs.

It was also revealed that the compounds of the present invention more strongly suppress HBV replication when used in combination with interferons or nucleic acid analogs. Thus, pharmaceutical compositions comprising the two ingredients can be used as a safer and more effective novel agent for treating HBV.

In addition, the present invention demonstrates that, when used in combination with interferons and/or nucleic acid analogs, the compound represented by formula (I) can also be used as a novel pharmaceutical composition for treating or preventing HBV infection, which is also effective against viruses resistant to drugs such as nucleic acid analogs.

## Claims

1. A pharmaceutical composition for treating or preventing HBV infection, which comprises as an active ingredient a compound represented by: formula (I): 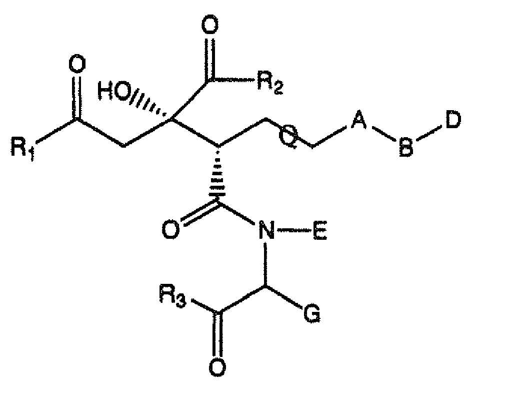 or a pharmaceutically acceptable salt thereof,;
wherein
A represents -(CH₂)ₙ-; wherein n represents an integer from 0 to 10;
B represents -CH₂-, -(C=O)-, -CH(OH)-, -CH(NH₂)-, or -C(=NOR)-; wherein R represents a hydrogen atom, a linear or branched alkyl group of 1 to 8 carbon atoms, which is optionally substituted with an amino group that is optionally mono- or di-substituted with a linear or branched alkyl group of 1 to 4 carbon atoms;
D represents -(CH₂)ₘ-R'; wherein m represents an integer from 0 to 10; R' represents a hydrogen atom, a linear or branched alkyl group, a linear or branched alkynyl group, a linear or branched alkenyl group, a cycloalkyl group, a cycloalkenyl group, an optionally substituted heterocyclic group, an optionally substituted aryl group, an optionally substituted heteroaryl group, -OX group (wherein X represents a hydrogen atom, a linear or branched alkyl group, a linear or branched alkynyl group, a linear or branched alkenyl group, a cycloalkyl group, or an optionally substituted aryl group), or a halogen atom;
E represents a hydrogen atom or a linear or branched alkyl group;
G represents -(CH₂)ₚ-J; wherein p represents an integer from 0 to 4; J represents a hydrogen atom, an OH group, an SH group, a methylthio group, a carboxyl group, a carbamoyl group, an amino group, a guanidino group, a linear or branched alkyl group, a cycloalkyl group, a linear or branched alkynyl group, a linear or branched alkenyl group, an optionally substituted aryl group, an optionally substituted heterocyclic group, or an optionally substituted heteroaryl group;
bond Q represents a single bond or double bond; and
R¹, R², and R³ are the same or different and each represents a hydroxyl group, an amino group which is optionally mono- or di-substituted by a linear or branched alkyl group having 1 to 4 carbon atoms, -OL, a linear or branched alkyl group, a linear or branched alkenyl group, or a linear or branched alkynyl group; wherein L represents a linear or branched alkyl group, a linear or branched alkenyl group, or a linear or branched alkynyl group.

2. The pharmaceutical composition of claim 1, wherein the compound is represented by any one of: 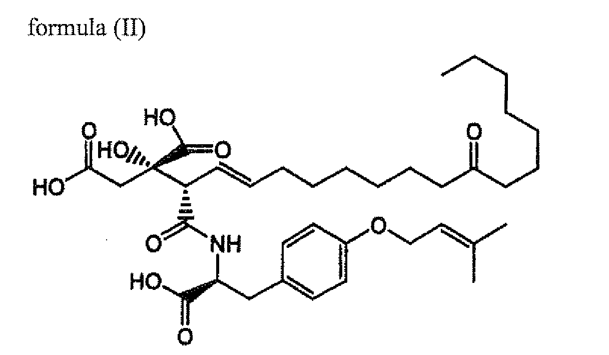 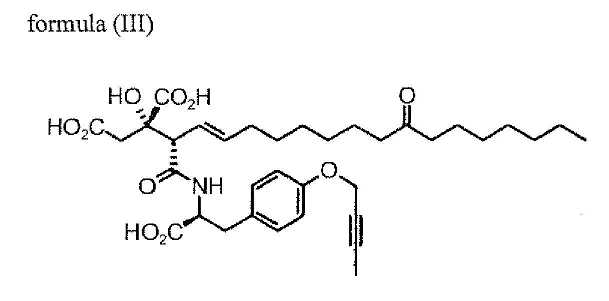 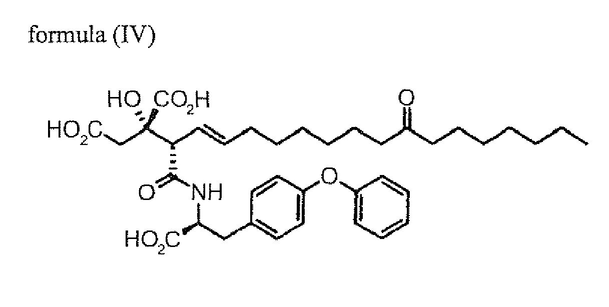 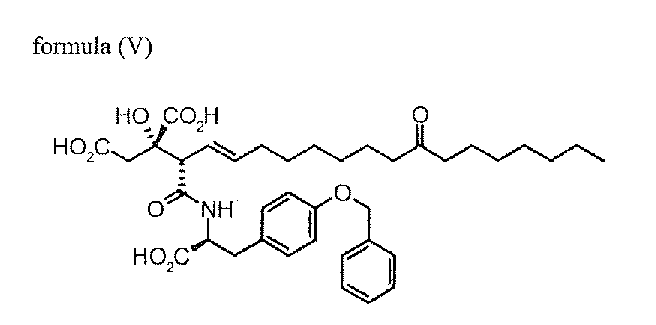 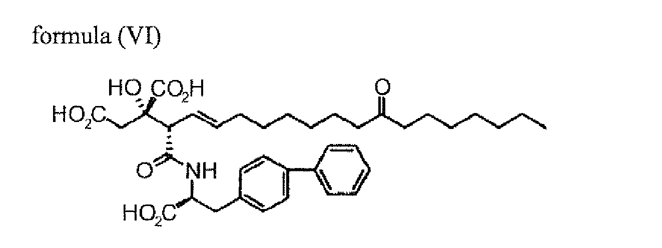 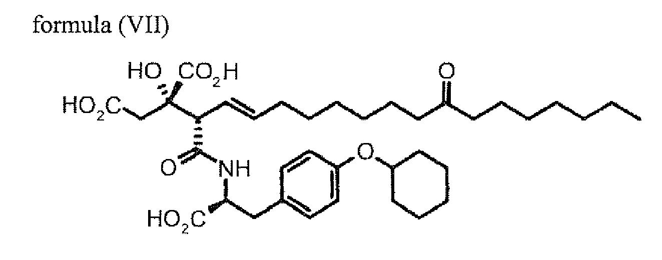 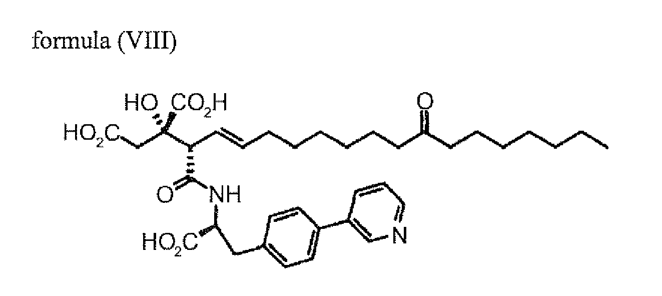 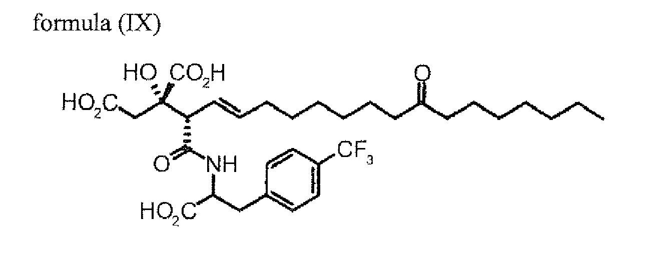 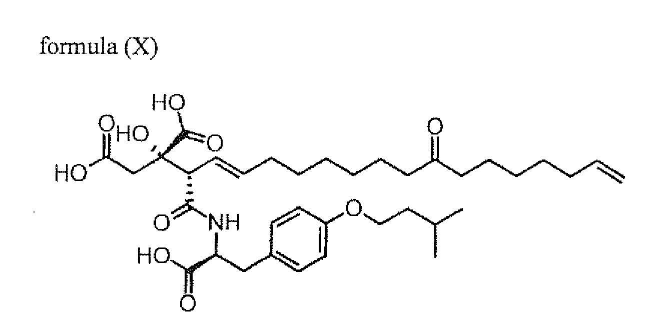 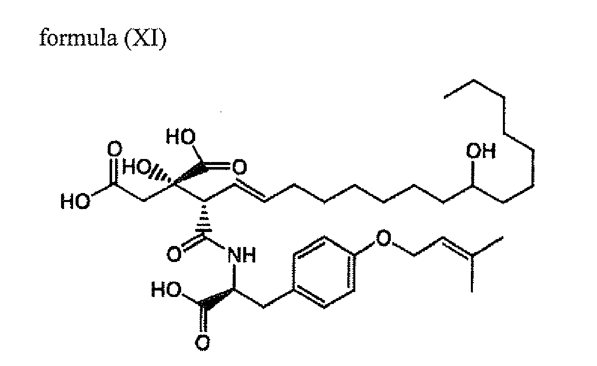 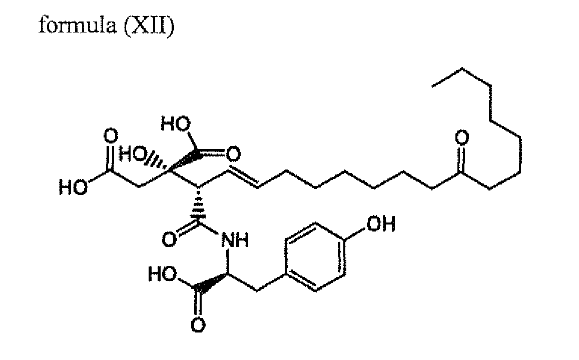 or a pharmaceutically acceptable salt thereof.

3. The pharmaceutical composition of claim 1 or 2, wherein the HBV infection is hepatitis B, cirrhosis, or liver cancer.

4. A method for treating or preventing HBV infection, which comprises the step of administering at a therapeutically effective dose a compound represented by formula (I) in claim 1, or a pharmaceutically acceptable salt thereof, to a subject.

5. Use of a compound represented by formula (I) in claim 1 or a pharmaceutically acceptable salt thereof in preparation of a pharmaceutical composition for treating or preventing HBV infection.
